# EUROPEAN PATENT SPECIFICATION

(11) **EP 2 593 012 B1**
(45) Date of publication and mention of the grant of the patent: **17.03.2021**
(21) Application number: 11806963.2
(22) Date of filing: 13.04.2011
(51) Int. Cl.: A61B 5/145, A61B 5/11, A61B 5/157

(54) **BLOOD GLUCOSE MEASUREMENT METHOD USING BLOOD GLUCOSE METER HAVING STEP COUNTER FUNCTION**
BLUTZUCKERMESSVERFAHREN MIT EINEM BLUTZUCKERMESSGERÄT MIT SCHRITTZÄHLERFUNKTION
PROCÉDÉ DE MESURE DE GLYCÉMIE À L'AIDE D'UN GLUCOMÈTRE AYANT UNE FONCTION DE COMPTEUR D'ÉTAPES

(30) Priority: 13.07.2010 KR 20100067202
(43) Date of publication of application: 22.05.2013
(73) Proprietor: Philosys Co., Ltd., Gunsan-si, Jeollabuk-do 573-708 (KR)
(72) Inventor: CHOI, In Hwan, Suwon Si Gyeonggi-Do 442-190 (KR); BAG, Jeong In, Anyang Si Gyeonggi-Do 431-745 (KR); KIM, Su Hwan, Seoul 122-020 (KR); PYO, Ji Hoon, Seoul 135-230 (KR)
(74) Representative: Long, Giorgio
(86) International application number: PCT/KR2011/002625
(87) International publication number: WO 2012/008678

(56) References cited:
- JP-A- 2006 110 211
- KR-A- 20060 121 476
- KR-A- 20060 125 055
- KR-A- 20090 004 040
- US-A1- 2006 173 260
- US-A1- 2009 221 890
- US-A1- 2009 264 337

## Description

### Technical Field

The prevent invention relates, in general, to a blood glucose measurement method using a blood glucose meter having a step counter function, and more particularly, to a blood glucose measurement method using a blood glucose meter having a step counter function, which can selectively measure the amount of exercise and a blood glucose level by combining the function of a blood glucose meter with the function of a step counter.

### Background Art

Generally, blood glucose (blood sugar) denotes the concentration of glucose in the blood, and the blood glucose level must always be kept within a certain range so as to maintain a human body in homeostasis.

Hormones attributing to the maintenance of the blood glucose level include glucagon, adrenalin, insulin, thyroid hormone, etc., and the blood glucose level of a human body is always maintained at about 90 mg/dl by the antagonism between such hormones.

When an abnormality occurs in the secretion of hormones required to maintain the blood glucose level, the blood glucose level becomes unstable. Diabetes is the disease appearing when an abnormality occurs specifically in the secretion of insulin which functions to decrease the blood glucose level.

Medication is important in the treatment of diabetes, but it is also important to prevent complications in such a way as to continuously measure the blood glucose level and acquire information about diabetes by performing eating based on alimentotherapy and continuously doing physical exercise.

Aerobic exercises that use more oxygen are effective as the exercise suitable for diabetes, and, in particular, the exercise of walking is well-known as a good exercise.

However, in the case of diabetics, excessive exercise may cause hypoglycemia.

Hypoglycemia refers to the case where the blood glucose level decreases to 50 mg/dl or less. When hypoglycemia exists, symptoms such as a sense of hunger, tremors in the hands and feet, dizziness, mental disorders, and an entire body convulsion occur. In a serious case, a patient may enter a coma or die, and thus there is a need to check and manage the amount of exercise and the blood glucose level at all times while doing exercise.

Therefore, a patient puts on a step counter so as to measure the amount of exercise, and exercises while carrying a blood glucose meter so as to check the blood glucose level before and after doing exercise. This method suffers from the inconvenience of having to separately carry the step counter and the blood glucose meter when exercising, and the method also has the difficulty of having to analyze the relationship between the amount of exercise measured by the step counter and the blood glucose level measured by the blood glucose meter. Further, a problem arises in that since aged persons are poor in their ability to analyze the relationship between the amount of exercise and the blood glucose level, they avoid exercising due to anxiety about hypoglycemia. US2009/264337 describes a glucometer for blood glucose monitoring, a pedometer for exertion measurement, combined with user-entered dietary or other diabetes-relevant information. US2006/173260 discloses a diabetes monitoring device that may include sensors for monitoring one or more of for example blood glucose level, physical activity, energy intake and insulin dosage. US2009/221890 describes a glucose monitoring system controlled by a touch-screen remote device.

### Disclosure of Invention

### Technical Problem

Accordingly, the present invention has been made keeping in mind the above problems occurring in the prior art, and an object of the present invention is to provide a blood glucose measurement method using a blood glucose meter having a step counter function, which can selectively measure the amount of exercise and the blood glucose level by combining the function of a blood glucose meter with the function of a step counter.

Another object of the present invention is to provide a blood glucose measurement method using a blood glucose meter having a step counter function, which provides an estimated blood glucose level by comparatively analyzing blood glucose level data measured in blood glucose measurement mode and the amount of exercise measured in step counting operation mode, thus facilitating the management of blood glucose, and which anticipates hypoglycemia, thus eliminating the psychological anxiety that causes exercise to be avoided.

### Solution to Problem

In order to accomplish the above objects, the present invention provides a blood glucose measurement method using a blood glucose meter having a step counter function, including displaying a selection window for blood glucose measurement mode and step counting operation mode; selecting the blood glucose measurement mode from the selection window; measuring a blood glucose level in the blood glucose measurement mode; configuring the measured blood glucose level in a form of data, storing the data in a control unit, and displaying the measured blood glucose level on a display unit under control of the control unit; selecting the step counting operation mode from the selection window; counting a number of steps taken by a user in the step counting operation mode; and storing a count result in the control unit and displaying the count result on the display unit, wherein a blood glucose level and an amount of exercise are individually measured depending on selection of the mode.

Further, the present invention provides a blood glucose measurement method using a blood glucose meter having a step counter function, including measuring a blood glucose level in blood glucose measurement mode; configuring the measured blood glucose level in a form of data, storing the data in a control unit, and displaying the measured blood glucose level on a display unit under control of the control unit; switching current mode to step counting operation mode; and counting a number of steps taken by a user in the step counting operation mode and displaying a count result on the display unit.

Preferably, the blood glucose measurement method may further include, after the counting the number of steps, calculating a variation in blood glucose relative to the amount of exercise using the count result corresponding to the number of steps; calculating an estimated blood glucose level by comparing the calculated blood glucose variation with previously measured blood glucose level data; and displaying the estimated blood glucose level on the display unit.

Preferably, the calculating the estimated blood glucose level may include determining whether the estimated blood glucose level falls outside a preset range of safe numerical values, and displaying a warning output while outputting a beeping sound if it is determined that the estimated blood glucose level falls outside the range of the safe numerical values.

Preferably, each numeral in the blood glucose measurement mode may be displayed in a form of a numeral of 3 or fewer digits, and each numeral in the step counting operation mode may be displayed in a form of a numeral of 5 or fewer digits.

Preferably, the step counting operation mode may be implemented using an electromechanical switch or a piezoelectric type-sensor which counts the number of steps taken by the user.

### Advantageous Effects of Invention

Accordingly, the blood glucose measurement method using a blood glucose meter having a step counter function in accordance with the present invention is advantageous in that the amount of exercise and the blood glucose level can be selectively measured by the blood glucose meter, thus enabling the present invention to be very conveniently used, and in that blood glucose level data, measured in blood glucose measurement mode before doing exercise, and a variation in the blood glucose relative to the amount of exercise, measured in step counting operation mode, are comparatively analyzed, so that the blood glucose level that can be estimated after doing exercise is provided, thus facilitating the management of blood glucose and eliminating the psychological anxiety that causes exercise to be avoided thanks to hypoglycemia being able to be anticipated beforehand.

### Brief Description of Drawings

FIG. 1 is a system configuration diagram showing a blood glucose measurement method using a blood glucose meter having a step counter function according to the present invention;
FIG. 2 is a flowchart showing a blood glucose measurement method using a blood glucose meter having a step counter function according to an embodiment of the present invention;
FIG. 3 is a flowchart showing a blood glucose measurement method using a blood glucose meter having a step counter function according to another embodiment of the present invention;
FIG. 4A and 4B are diagrams showing a numerical display unit operated in blood glucose measurement mode and step counting operation mode, respectively, in the blood glucose measurement method using a blood glucose meter having a step counter function according to the present invention; and
FIG. 5 is a diagram showing a display unit at step 5270 in the blood glucose measurement method using a blood glucose meter having a step counter function according to the present invention.

### Best Mode for Carrying out the Invention

The above and other objects, features and advantages of the present invention will be more clearly understood from the following detailed description taken in conjunction with the accompanying drawings. Hereinafter, a blood glucose measurement method using a blood glucose meter having a step counter function according to embodiments of the present invention will be described in detail with reference to the attached drawings. For the sake of the present specification, the same reference numerals are used throughout the different drawings to designate the same or similar components unless a description thereof is especially made.

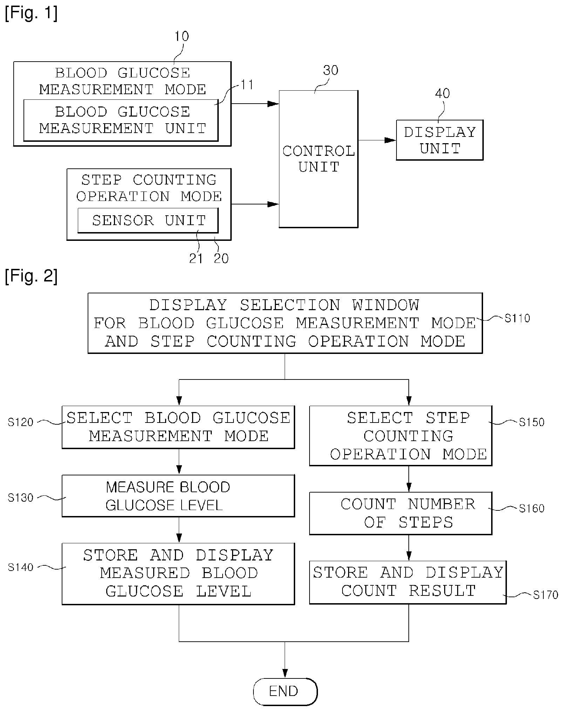

As shown in FIGS. 1 and 2, an embodiment of the present invention includes steps S110 to S170. At step S110, a selection window for blood glucose measurement mode and step counting operation mode is displayed. At step S120, the blood glucose measurement mode is selected from the selection window. At step 5130, the blood glucose level is measured in the blood glucose measurement mode. At step S140, the measured blood glucose level is configured in the form of data and is stored in a control unit 30, and the measured blood glucose level is displayed on a display unit 40 under the control of the control unit 30. At step S150, the step counting operation mode is selected from the selection window. At step S160, the number of steps taken by a user is counted in the step counting operation mode. At step S170, the count result is stored in the control unit 30 and is displayed on the display unit 40. Accordingly, depending on the selection of the mode, the blood glucose level and the amount of exercise can be separately measured.

In this case, when power is applied to the blood glucose meter, the selection window for blood glucose measurement mode 10 and step counting operation mode 20 is displayed. When the blood glucose measurement mode is selected from the displayed selection window, the blood glucose level can be measured. Further, when the user selects the step counting operation mode 20 from the selection window, the amount of exercise can be measured.

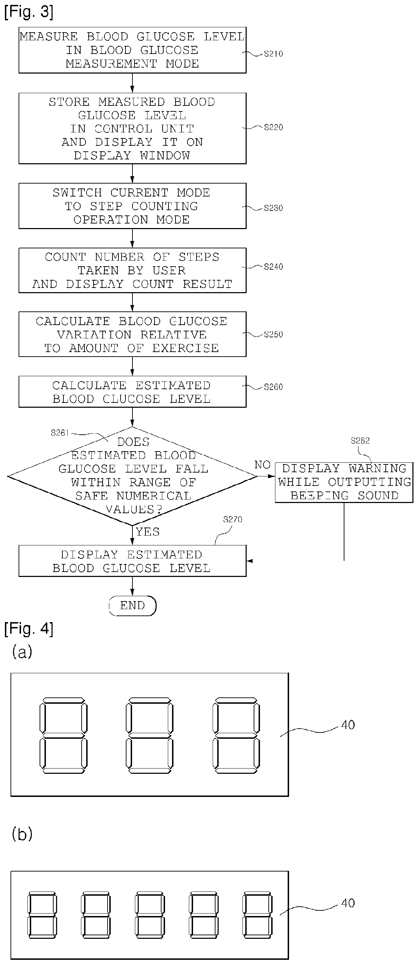

As shown in FIGS. 1 and 3, another embodiment of the present invention includes steps 5210 to S270. At step 5210, the blood glucose level is measured in the blood glucose measurement mode 10. At step 5220, the measured blood glucose level is configured in the form of data and is stored in the control unit 30, and the measured blood glucose level is displayed on the display unit 40 under the control of the control unit 30. At step 5230, current mode is switched to step counting operation mode 20. At step 5240, the number of steps taken by the user is counted in the step counting operation mode, and the control unit 30 receives the count result and displays it on the display unit 40. At step 5250, the control unit 30 calculates a variation in the blood glucose relative to the amount of exercise using the count result. At step 5260, the calculated blood glucose variation is compared to the previously measured blood glucose level data, and then an estimated blood glucose level is calculated. At step S270, the estimated blood glucose level is displayed on the display unit 40.

In this case, when power is applied to the blood glucose meter, a window for the blood glucose measurement mode 10 is displayed first.

Step S130 or 5210 is the step where the blood glucose level is measured in the window for the blood glucose measurement mode 10, and is configured to supply blood to a blood glucose measurement unit 11. Methods of supplying blood include a method of directly dropping blood down onto the blood glucose measurement unit 11 and a method of absorbing blood using a blood glucose strip (test paper) or the like. The blood glucose strip (test paper) which absorbs the blood needs only to be inserted into a blood glucose strip slot formed on the blood glucose measurement unit 11.

When blood is supplied to the blood glucose measurement unit 11, the blood glucose measurement unit 11 recognizes the blood, measures the blood glucose level, and outputs the measured blood glucose level to the control unit 30.

Step 5140 or 5220 is configured such that the control unit 30 receives the measured blood glucose level output from the blood glucose measurement unit 11, configures it in the form of data, stores the data, digitizes the measured blood glucose level into a numerical value, and displays the numerical value on the display unit 40.

As shown in FIGS. 4A and 4B, the display unit 40 is implemented as a matrix-type Liquid Crystal Display (LCD) and variably displays numerals of 3 to 5 digits, wherein each numeral in the blood glucose measurement mode is displayed in the form of a numeral of 3 or fewer digits, as shown in FIG. 4A.

Step 5230 is configured such that when a predetermined period of time has elapsed after the measured blood glucose level was displayed, the current mode is switched to step counting operation mode 20. In this case, the switching of the step counting operation mode 20 may be performed in such a way that the selection window for the blood glucose measurement mode 10 and the step counting operation mode 20 is displayed again to allow the user to select the step counting operation mode 20 or in such a way that the current mode is directly switched to the step counting operation mode 20.

Step S160, S170 or 5240 is configured such that whenever the user walks, a sensor unit 21 counts the number of steps taken by the user and outputs a count result to the control unit 30. Further, the control unit 30 displays the count result on the display unit 40. In this case, as shown in FIG 4B, each numeral in the step counting operation mode 20 is displayed in the form of a numeral of 5 or fewer digits.

Here, the sensor unit 21 must be implemented as a low-power sensor due to the characteristics of the blood glucose meter. Therefore, an electromechanical switch or a piezoelectric type-sensor which uses low power, rather than an acceleration sensor, may preferably be used. The reason for this is that the low-power sensor has a simpler circuit construction than the acceleration sensor and can implement a desired operation using a circuit with lower-current consumption.

Step 5250 is configured such that the control unit 30 calculates a variation in blood glucose relative to the amount of exercise based on the count result. Further, the amount of exercise may be converted into calories, so that the blood glucose variation relative to the calorie consumption may also be calculated. In this case, in the control unit 30, information about the blood glucose variation relative to the amount of exercise or calorie consumption is set in advance.

Step 5260 is configured to compare the calculated blood glucose variation with the blood glucose level data previously measured in the blood glucose measurement mode 10. That is, the current estimated blood glucose level can be calculated by subtracting the blood glucose variation relative to the amount of exercise from the blood glucose level data previously measured in the blood glucose measurement mode 10 before doing exercise.

Next, after step 5260, it is determined whether the estimated blood glucose level falls outside a preset range of safe numerical values at step 5261. If it is determined that the estimated blood glucose level falls within the preset range of the safe numerical values, the estimated blood glucose level is displayed in the form of a numerical value on the display unit 40 at step 5270. In contrast, if it is determined that the estimated blood glucose level falls outside the preset range of the safe numerical values, a warning output is displayed on the display unit 40 while a beeping sound is output at step 5262. In addition, the estimated blood glucose level is displayed on the display unit 40 in the form of a numerical value at step 5270.

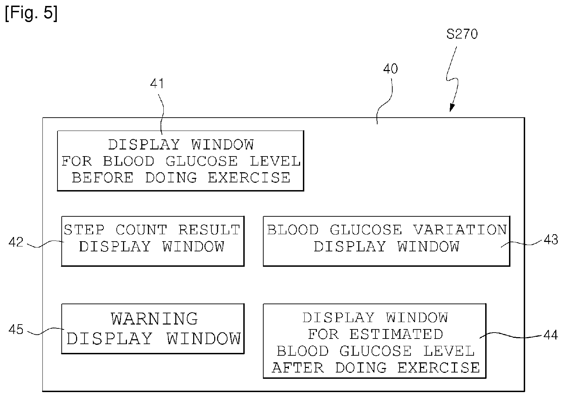

Here, step 5270 is configured to display the estimated blood glucose level after doing exercise. In this case, only the estimated blood glucose level may be displayed. Alternatively, as shown in FIG. 5, step 5270 may be preferably performed such that all of a display window 41 for displaying the blood glucose level measured before doing exercise, a display window 42 for displaying the count result (the amount of exercise) corresponding to the number of steps taken by the user, a display window 43 for displaying a blood glucose variation, a display window 44 for displaying the current estimated blood glucose level after doing exercise, a warning display window 45, etc. are displayed, thus allowing the user to simultaneously monitor the relationship between the amount of exercise and the blood glucose variation and the relationship between the blood glucose levels obtained before and after doing exercise.

Although the preferred embodiments of the present invention have been disclosed for illustrative purposes, those skilled in the art will appreciate that various modifications and changes are possible, without departing from the scope of the invention whose scope is defined in the accompanying claims.

### Industrial Applicability

As described above, the present invention is advantageous in that since the amount of exercise and the blood glucose level can be selectively measured by a blood glucose meter, the present invention can be very conveniently used. Further, the present invention is advantageous in that blood glucose level data, measured in blood glucose measurement mode before doing exercise, and a blood glucose variation relative to the amount of exercise, measured in step counting operation mode, are comparatively analyzed, so that an estimated blood glucose level is provided, thus enabling the management of blood glucose to be possible even if a separate blood glucose measurement is not made after doing exercise. Furthermore, the present invention is advantageous in that hypoglycemia can be anticipated, thus eliminating the psychological anxiety that causes exercise to be avoided.

## Claims

1. A blood glucose measurement method using a blood glucose meter (11) having a step counter function, comprising:
measuring a blood glucose level in blood glucose measurement mode (10);
configuring the measured blood glucose level in a form of data, storing the data in a control unit (30), and displaying the measured blood glucose level on a display unit (40) under control of the control unit (30);
switching current mode to step counting operation mode (20);
counting a number of steps taken by a user in the step counting operation mode and displaying a count result on the display unit (40),
calculating a variation in blood glucose relative to an amount of exercise using the count result corresponding to the number of steps;
calculating an estimated blood glucose level by subtracting the calculated blood glucose variation relative to the amount of exercise from previously measured blood glucose level data; and
displaying the estimated blood glucose level on the display unit (40),
wherein the step counting operation mode is implemented using an electromechanical switch or a piezoelectric type-sensor which counts the number of steps taken by the user.

2. The blood glucose measurement method according to claim 1, wherein the calculating the estimated blood glucose level comprises determining whether the estimated blood glucose level falls outside a preset range of safe numerical values, and displaying a warning output while outputting a beeping sound if it is determined that the estimated blood glucose level falls outside the range of the safe numerical values.

3. The blood glucose measurement method according to claim 1, wherein each numeral in the blood glucose measurement mode (10) is displayed in a form of a numeral of 3 or fewer digits, and each numeral in the step counting operation mode is displayed in a form of a numeral of 5 or fewer digits.

## Patentansprüche

1. Verfahren zur Blutzuckermessung unter Verwendung eines Blutzuckermessgerätes (11) mit einer Schrittzählerfunktion, umfassend:
Messen eines Blutzuckerspiegels im Blutzuckermessmodus (10);
Konfigurieren des gemessenen Blutzuckerspiegels in einer Datenform, Speichern der Daten in einer Steuereinheit (30) und Anzeigen des gemessenen Blutzuckerspiegels auf einer Anzeigeeinheit (40) unter Steuerung der Steuereinheit (30);
Umschalten des aktuellen Modus in den Schrittzählbetriebsmodus (20);
Zählen einer Anzahl von Schritten, die von einem Benutzer in dem Schrittzählbetriebsmodus gemacht werden, und Anzeigen eines Zählergebnisses auf der Anzeigeeinheit (40),
Berechnen einer Veränderung des Blutzuckers im Verhältnis zu einem Ausmaß an Bewegung unter Verwendung des Zählergebnisses, das der Anzahl von Schritten entspricht;
Berechnen eines geschätzten Blutzuckerspiegels durch Subtrahieren der berechneten Blutzuckerveränderung im Verhältnis zu dem Ausmaß an Bewegung von zuvor gemessenen Blutzuckerspiegeldaten; und
Anzeigen des geschätzten Blutzuckerspiegels auf der Anzeigeeinheit (40),
wobei der Schrittzählerbetriebsmodus unter Verwendung eines elektromechanischen Schalters oder eines piezoelektrischen Sensortyps implementiert ist, der die Anzahl der Schritte zählt, die von dem Benutzer gemacht werden.

2. Blutzuckermessverfahren nach Anspruch 1, wobei das Berechnen des geschätzten Blutzuckerspiegels das Bestimmen umfasst, ob der geschätzte Blutzuckerspiegel außerhalb eines voreingestellten Bereichs von sicheren numerischen Werten liegt, und Anzeigen einer Warnausgabe unter Ausgabe eines Pieptons, wenn bestimmt wird, dass der geschätzte Blutzuckerspiegel außerhalb des Bereichs der sicheren numerischen Werte fällt.

3. Blutzuckermessverfahren nach Anspruch 1, wobei jede Zahl in dem Blutzuckermessmodus (10) in Form einer Zahl mit 3 oder weniger Ziffern angezeigt wird, und jede Zahl im Schrittzählbetriebsmodus in Form einer Zahl mit 5 oder weniger Ziffern angezeigt wird.

## Revendications

1. Procédé de mesure de la glycémie à l'aide d'un glucomètre (11) possédant une fonction de compteur de pas, comprenant :
la mesure d'un taux de glycémie en mode de mesure de la glycémie (10) ;
la configuration du taux de glycémie mesuré sous forme de données, le stockage des données dans une unité de contrôle (30), et l'affichage du taux de glycémie mesuré sur une unité d'affichage (40) sous le contrôle de l'unité de contrôle (30) ;
le passage du mode courant au mode de fonctionnement de compteur de pas (20) ;
le comptage d'un nombre de pas effectués par un utilisateur en mode de fonctionnement de compteur de pas et l'affichage d'un résultat de comptage sur l'unité d'affichage (40),
le calcul d'une variation de la glycémie par rapport à une quantité d'exercice en utilisant le résultat de comptage correspondant au nombre de pas ;
le calcul d'un taux de glycémie estimé en soustrayant la variation de la glycémie calculée par rapport à la quantité d'exercice des données relatives au taux de glycémie mesuré précédemment ; et
l'affichage du taux de glycémie estimé sur l'unité d'affichage (40),
dans lequel le mode de fonctionnement de compteur de pas est mis en œuvre en utilisant un commutateur électromécanique ou un capteur de type piézoélectrique qui compte le nombre de pas effectués par l'utilisateur.

2. Procédé de mesure de la glycémie selon la revendication 1, dans lequel le calcul du taux de glycémie estimé comprend la détermination pour savoir si le taux de glycémie estimé se situe en dehors d'une plage prédéfinie de valeurs numériques sûres, et l'affichage d'un avertissement tout en émettant un bip sonore s'il est établi que le taux de glycémie estimé se situe en dehors de la plage des valeurs numériques sûres.

3. Procédé de mesure de la glycémie selon la revendication 1, dans lequel chaque chiffre dans le mode de mesure de la glycémie (10) est affiché sous la forme d'un nombre à 3 chiffres ou moins, et chaque chiffre dans le mode de fonctionnement de compteur de pas est affiché sous la forme d'un nombre à 5 chiffres ou moins.
